# EUROPEAN PATENT APPLICATION

(11) **EP 4 442 829 A1**
(43) Date of publication of application: **09.10.2024**
(21) Application number: 22916709.3
(22) Date of filing: 27.12.2022
(51) Int. Cl.: C12N 15/70, C12N 9/04, C12P 13/06

(54) **O-PHOSPHOSERINE-PRODUCING MICROORGANISM, AND METHOD FOR PRODUCING O-PHOSPHOSERINE OR L-CYSTEINE BY USING SAME**

(30) Priority: 31.12.2021 KR 20210193961
(71) Applicant: CJ CheilJedang Corporation, Seoul 04560 (KR)
(72) Inventor: JUNG, Hwi-Min, Seoul 04560 (KR); RHO, Jin Ah, Seoul 04560 (KR); SIM, Hee-jin, Seoul 04560 (KR); PARK, Hye Min, Seoul 04560 (KR)
(74) Representative: Mewburn Ellis LLP
(86) International application number: PCT/KR2022/021420
(87) International publication number: WO 2023/128568

(57) **Abstract**

The present application relates to a microorganism in which an activity of erythronate-4-phosphate dehydrogenase is weakened; a method for producing *O-*phosphoserine, cysteine, or cysteine derivatives using the microorganism; a composition for producing *O*-phosphoserine comprising the microorganism; and the use of the microorganism in the production of *O*-phosphoserine, cysteine, and cysteine derivatives.

## Description

### [Technical Field]

The present application relates to a microorganism in which the activity of erythronate-4-phosphate dehydrogenase is weakened; a method for producing *O-*phosphoserine, cysteine, or cysteine derivatives using the microorganism; a composition for producing *O*-phosphoserine comprising the microorganism; and the use of the microorganism in the production of *O*-phosphoserine, cysteine, and cysteine derivatives.

### [Background Art]

L-Cysteine, an amino acid having an important role in sulfur metabolism in all living organisms, is used not only in the synthesis of biological proteins such as hair keratin, glutathione, biotin, methionine, and other sulfur-containing metabolites, but also as a precursor for biosynthesis of coenzyme A.

Methods of producing L-cysteine using microorganisms known in the art include: 1) a method of biologically converting D,L-2-aminothiazoline-4-carboxylic acid (D,L-ATC) into L-cysteine using microorganisms, 2) a method of producing L-cysteine by direct fermentation using *E*. *coli* (EP 0885962 B; Wada M and Takagi H, Appl. Microbiol. Biochem., 73:48-54, 2006), and 3) a method of producing *O-*phosphoserine (hereinafter "OPS") by fermentation using microorganisms, and then converting *O*-phosphoserine into L-cysteine by reacting *O*-phosphoserine with a sulfide under the catalytic action of *O*-phosphoserine sulfhydrylase (hereinafter, "OPSS") (US 8557549 B2).

In particular, in order to produce cysteine by way of method 3) with high yield, OPS, the precursor, should be produced in an excess amount.

### [Disclosure]

### [Technical Problem]

The present inventors have developed an *O*-phosphoserine-producing microorganism, in which an activity of erythronate-4-phosphate dehydrogenase is weakened, a method for producing *O*-phosphoserine using the microorganism, a method for producing cysteine or a derivative thereof using the microorganism, and an *O*-phosphoserine-producing composition comprising the microorganism, thereby completing the present disclosure.

### [Technical Solution]

It is one object of the present application to provide an *O*-phosphoserine-producing microorganism, in which an activity of erythronate-4-phosphate dehydrogenase is weakened.

It is another object of the present application to provide a method for producing *O*-phosphoserine, including culturing the microorganism of the present application, in a medium.

It is still another object of the present application to provide a method for producing cysteine or a derivative thereof, including:
a) culturing the microorganism of the present application in a medium to produce *O*-phosphoserine or a medium containing the same; and b) reacting O-phosphoserine sulfhydrylase (OPSS) or a microorganism expressing the same; O-phosphoserine produced in step a) or a medium containing the same; and a sulfide.
It is yet another object of the present application to provide a composition for producing *O*-phosphoserine, including the microorganism of the present application.
It is even another object of the present application to provide the use of the microorganism of the present application for the production of *O*-phosphoserine, cysteine, or cysteine derivatives.

### [Advantageous Effects]

The present application can produce *O*-phosphoserine with high efficiency using an *O*-phosphoserine-producing microorganism in which the activity of erythronate-4-phosphate dehydrogenase is weakened compared to its endogenous activity. In addition, when cysteine and derivatives thereof are produced through enzymatic conversion of *O*-phosphoserine produced above, cysteine can be produced in a more environmentally friendly and highly efficient manner than chemical synthesis.

### [DETAILED DESCRIPTION OF THE INVENTION]

The present application will be described in detail. Meanwhile, each description and embodiment disclosed herein can be applied to other descriptions and embodiments, respectively. That is, all combinations of various elements disclosed herein fall within the scope of the present application. Further, the scope of the present application is not limited by the specific description described below.

Additionally, a number of papers and patent documents have been cited throughout the present specification. The contents of the cited papers and patent documents are incorporated herein by reference in their entirety, and the level of technical field to which the present application belongs and the contents of the present application will be described more clearly.

One aspect of the present application provides an *O*-phosphoserine-producing microorganism, in which an activity of erythronate-4-phosphate dehydrogenase is weakened.

As used herein, the term "erythronate-4-phosphate dehydrogenase" is an enzyme that oxidizes erythronate-4-phosphate into 3-hydroxy-4-phospho-hydroxy-α-ketobutyrate in the pyridoxine biosynthetic pathway of microorganisms, and in the present application, the erythronate-4-phosphate dehydrogenase may be used interchangeably with "PdxB". Specifically, the erythronate-4-phosphate dehydrogenase is known in the art, and the protein and gene sequences of the erythronate-4-phosphate dehydrogenase can be obtained from a known database, such as NCBI GenBank, but is not limited thereto. More specifically, the erythronate-4-phosphate dehydrogenase may have and/or include the amino acid sequence of SEQ ID NO: 1, or may consist essentially of or consist of the amino acid sequence.

The erythronate-4-phosphate dehydrogenase of the present application may include an amino acid sequence having a homology or identity of 70%, 75%, 80%, 85%, 90%, 95%, 96%, 97%, 98%, 99%, 99.5%, 99.7%, or 99.9% or more to the amino acid sequence of SEQ ID NO: 1. Additionally, it is apparent that any erythronate-4-phosphate dehydrogenase having an amino acid sequence, in which part of the sequence is deleted, modified, substituted, conservatively substituted, or added, may also fall within the scope of the present application as long as the amino acid sequence has such a homology or identity and exhibits an efficacy corresponding to that of the erythronate-4-phosphate dehydrogenase of the present application.

In the present application, although it is described as "a polypeptide or protein including an amino acid sequence of a particular SEQ ID NO", "a polypeptide or protein consisting of an amino acid sequence of a particular SEQ ID NO", or "a polypeptide or protein having an amino acid sequence of a particular SEQ ID NO", it is apparent that any protein which has deletion, modification, substitution, conservative substitution, or addition in part of the amino acid sequence may also be used in the present application, as long as the protein has the same or corresponding activity to the polypeptide consisting of the amino acid sequence of the corresponding SEQ ID NO. For example, it may be a case where the N-terminus and/or C-terminus of the amino acid sequence is added with a sequence that does not alter the function of the protein, a naturally occurring mutation, a potential mutation thereof, a silent mutation, or a conservative substitution.

For example, it may be a case where the N-terminus, C-terminus and/or inside of the amino acid sequence is added or deleted with a sequence that does not alter the function of the erythronate-4-phosphate dehydrogenase of the present application, a naturally occurring mutation, a potential mutation thereof, a silent mutation, or a conservative substitution.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Additionally, amino acids can be classified into amino acids with electrically charged side chains and amino acids with uncharged side chains, and examples of the amino acids with electrically charged side chains include aspartic acid, glutamic acid, lysine, arginine, and histidine. The amino acids with uncharged side chains can be further classified into nonpolar amino acids or polar amino acids. Examples of the non-polar amino acids are glycine, alanine, valine, leucine, isoleucine, methionine, phenylalanine, tryptophan, proline, and examples of the polar amino acids include serine, threonine, cysteine, tyrosine, asparagine, and glutamine. Typically, conservative substitutions have little or no effect on the activity of the resulting polypeptide. Typically, conservative substitutions may have little or no effect on the activity of the protein or polypeptide.

Additionally, the erythronate-4-phosphate dehydrogenase may also include deletion or addition of amino acids that have minimal influence on the properties and secondary structure of a polypeptide. For example, the polypeptide may be conjugated with a signal (or leader) sequence at the N-terminus involved in the transfer of proteins co-translationally or post-translationally. Further, the polypeptide may also be conjugated with another sequence or linker to identify, purify, or synthesize the polypeptide.

As used herein, the term "homology" or "identity" refers to a degree of relatedness between two given amino acid sequences or nucleotide sequences, and may be expressed as a percentage. The terms homology and identity may often be used interchangeably with each other.

The sequence homology or identity of conserved polynucleotide or polypeptide may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (*i.e*., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277), *etc.,* but is not limited thereto, and sequence alignment programs, such as pairwise sequence comparison algorithms, *etc.,* known in the art may be appropriately used.

As used herein, the term "*O*-phosphoserine" (OPS) refers to a phosphoric acid ester of serine which serves as a constituting component for many proteins. The OPS is a precursor of L-cysteine and can be converted to cysteine by reacting with a sulfide under the catalytic action of OPS sulfhydrylase (hereinafter, "OPSS"), but is not limited thereto (US 8557549 B2).

As used herein, the term "an *O*-phosphoserine-producing microorganism" refers to a microorganism that has a natural *O*-phosphoserine producing ability, or a microorganism that has been given an *O*-phosphoserine producing ability to a parent strain that does not have an *O*-phosphoserine producing ability. For the purpose of the present application, the *O*-phosphoserine-producing microorganism is characterized in that the activity of the PdxB protein is weakened and the desired *O-*phosphoserine producing ability is increased, and may be a genetically modified microorganism or a recombinant microorganism, but is not limited thereto.

As used herein, the term "strain" or "microorganism" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired polypeptide, protein or product.

The microorganism of the present application may be a microorganism having an O-phosphoserine producing ability.

The microorganism of the present application may be a microorganism that has a natural *O*-phosphoserine producing ability, or a microorganism that has been given an *O*-phosphoserine producing ability to a parent strain that does not have an *O*-phosphoserine producing ability, but is not limited thereto.

For example, the strain of the present application may be a recombinant strain, in which the PdxB protein activity is weakened compared to the endogenous activity in a natural wild-type microorganism or an *O*-phosphoserine-producing microorganism, and thus, the *O*-phosphoserine producing ability is increased. The recombinant strain with increased *O*-phosphoserine producing ability may be a microorganism with increased *O*-phosphoserine producing ability compared to a natural wild-type microorganism or an unmodified microorganism having an endogenous PdxB protein activity.

The microorganism of the present application may be a microorganism having an increased ability to produce *O*-phosphoserine compared to a microorganism in which an initiation codon of a polynucleotide encoding the erythronate-4-phosphate dehydrogenase is GTG or ATG, but is not limited thereto. In one example, the recombinant strain having an increased production ability may have an increased *O*-phosphoserine producing ability by about 1% or more, specifically about 1% or more, about 10% or more, about 20% or more, about 29% or more, about 30% or more, about 40% or more, about 50% or more, about 60% or more, about 70% or more, about 80% or more, about 90% or more, about 100% or more, about 101% or more, about 102% or more, about 103% or more, about 104% or more, about 105% or more, about 106% or more, or about 107% or more (the upper limit is not particularly limited, for example, about 300% or less, about 200% or less, about 150% or less, about 110% or less, about 109% or less, or about 108% or less) as compared to the *O*-phosphoserine producing ability of the parent strain before modification or a non-modified microorganism having endogenous activity of PdxB protein, but is not limited thereto, as long as it has an increased + value compared to the production ability of the parent strain before modification or non-modified microorganism. In another example, the recombinant strain having an increased production ability may have an increased *O*-phosphoserine producing ability by about 1.01 times or more, about 1.02 times or more, about 1.03 times or more, about 1.04 times or more, about 1.05 times or more, about 1.06 times or more, or about 1.07 times or more (the upper limit is not particularly limited, for example, about 10 times or less, about 5 times or less, about 3 times or less, about 2 times or less, or about 1.5 times or less) as compared to that of the parent strain before modification or non-modified microorganism, but is not limited thereto.

As used herein, the term "non-modified strain" does not exclude a strain containing a mutation that may occur naturally in a microorganism, and may refer to a wild-type strain or natural-type strain itself, or a strain before the trait is altered due to genetic modification caused by natural or artificial factors. For example, the non-modified microorganism may refer to a strain in which the activity of erythronate-4-phosphate dehydrogenase described herein is not weakened compared to its endogenous activity, or a strain before the activity of erythronate-4-phosphate dehydrogenase is weakened. The "non-modified strain" may be used interchangeably with "strain before modification", "microorganism before modification", "non-mutant strain", "non-modified strain", "non-mutant microorganism", or "reference microorganism".

In another embodiment of the present application, the microorganism of the present application may be a microorganism capable of producing *O*-phosphoserine, and is not limited by its type. The microorganism of the present application may be any prokaryotic or eukaryotic microorganism, and specifically a prokaryotic microorganism. The prokaryotic microorganism may include microbial strains belonging to the genus *Escherichia,* the genus *Erwinia*, the genus *Serratia*, the genus *Providencia*, the genus *Corynebacterium* and the genus *Brevibacterium*, and specifically a microorganism belonging to the genus *Escherichia,* and more specifically *Escherichia coli*, but is not limited thereto. In particular, in the case of the microorganism belonging to the genus *Escherichia,* OPS and L-serine can be produced through SerA, SerC, and SerB, which are enzymes of the biosynthetic pathway of L-serine (Ahmed Zahoor, Computational and structural biotechnology journal, Vol. 3, 2012 October; Wendisch, V. F. et al., Curr Opin Microbiol. 2006 Jun;9(3):268-74; Peters-Wendisch, P. et al., Appl Environ Microbiol. 2005 Nov;7 1(II):7 139-44.).

The "non-modified microorganism" may be a microorganism whose initiator codon of a polynucleotide encoding erythronate-4-phosphate dehydrogenase is ATG or GTG, but is not limited thereto. More specifically, the "non-modified microorganism" may be a microorganism including a polynucleotide consisting of SEQ ID NO: 2.

As used herein, the term "weakening" of the activity of a polypeptide is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the polypeptide activity itself is decreased or removed compared to the activity of the polypeptide originally possessed by a microorganism due to a mutation of the polynucleotide encoding the polypeptide; a case where the overall level of intracellular polypeptide activity and/or concentration (expression level) is decreased compared to a natural strain due to the inhibition of expression of the gene of the polynucleotide encoding the polypeptide, or the inhibition of translation into the polypeptide, *etc*.; a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression that the polypeptide activity is "inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity can be performed by any method known in the art, but the method is not limited thereto, and can be achieved by applying various methods well known in the art (*e.g*., Nakashima N. et al., Bacterial cellular engineering by genome editing and gene silencing. Int J Mol Sci. 2014;15(2):2773-2793; Sambrook et al. Molecular Cloning 2012; *etc*.).

Specifically, the weakening of the polypeptide activity of the present application may be achieved by:
1) deleting a part or all of the gene encoding the polypeptide;
2) modifying the expression regulatory region (expression regulatory sequence) such that the expression of the gene encoding the polypeptide is decreased;
3) modifying the amino acid sequence constituting the polypeptide such that the polypeptide activity is removed or weakened (*e.g*., deletion/substitution/addition of one or more amino acids on the amino acid sequence);
4) modifying the gene sequence encoding the polypeptide such that the polypeptide activity is removed or weakened (*e.g*., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of the gene transcript encoding the polypeptide;
6) introducing an antisense oligonucleotide (*e.g*., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide; or
9) a combination of two or more selected from the methods 1) to 8) above,
but is not particularly limited thereto.

For example:
The 1) method of deleting a part or all of the gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome binding site, and a sequence for regulating the termination of transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto. For example, the expression of the gene may be inhibited or weakened by introducing a mutation into the polynucleotide sequence to form a termination codon, but is not limited thereto.

Further, the 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (*e.g*., antisense RNA), which binds complementary to the gene transcript encoding the polypeptide can be found in the literature (Weintraub, H. et al., Antisense-RNA as a molecular tool for genetic analysis, Reviews - Trends in Genetics, Vol. 1(1) 1986).

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of the gene sequence encoding the polypeptide may be achieved by forming an antisense nucleotide complementary to the gene transcript encoding the polypeptide to weaken the activity.

Specifically, the O-phosphoserine-producing microorganism of the present application may be one in which the activity of erythronate-4-phosphate dehydrogenase is weakened compared to its endogenous activity, but is not limited thereto.

Specifically, in order to weaken the activity of erythronate-4-phosphate dehydrogenase, the initiation codon of a gene transcript encoding a polypeptide may be modified.

As used herein, the term "initiation codon" refers to three nucleotides corresponding to the start point of translation when a coding sequence of messenger RNA (mRNA) is translated into a protein.

In the present application, the microorganism of the present application may be a microorganism whose initiation codon of a polynucleotide encoding the erythronate-4-phosphate dehydrogenase is TTG or CTG, but is not limited thereto.

As used herein, the term "polynucleotide", which is a polymer of nucleotides composed of nucleotide monomers connected in a lengthy chain by a covalently bond, is a DNA or RNA strand having at least a certain length. More specifically, it may refer to a polynucleotide fragment encoding the variant.

More specifically, the microorganism of the present application may be a microorganism including a polynucleotide consisting of SEQ ID NO: 3 and SEQ ID NO: 4.

The polynucleotide of the present application may undergo various modifications in the coding region within the scope that does not change the amino acid sequence of the variant of the present application, due to codon degeneracy or in consideration of the codons preferred in an organism in which the variant of the present application is to be expressed. Specifically, the polynucleotide of the present application may have or include a nucleotide sequence having a homology or identity of 70% or more, 75% or more, 80% or more, 85% or more, 90% or more, 95% or more, 96% or more, 97% or more, 98% or more, and 100% or less with the sequence of SEQ ID NO: 2, SEQ ID NO: 3, or SEQ ID NO: 4, or may consist or consist essentially of a nucleotide sequence having a homology or identity of 70% or more, 75% or more, 80% or more, 85% or more, 90% or more, 95% or more, 96% or more, 97% or more, 98% or more, and 100% or less with the sequence of SEQ ID NO: 2, SEQ ID NO: 3, or SEQ ID NO: 4, but is not limited thereto.

Additionally, the polynucleotide of the present application may include a probe that may be prepared from a known gene sequence, for example, any sequence which can hybridize with a sequence complementary to all or part of the polynucleotide sequence of the present application under stringent conditions without limitation. The "stringent conditions" refers to conditions under which specific hybridization between polynucleotides is allowed. Such conditions are specifically described in the literature (J. Sambrook et al., Molecular Cloning, A Laboratory Manual, 2nd Edition, Cold Spring Harbor Laboratory press, Cold Spring Harbor, New York, 1989; Ausubel, F. M. et al., Current Protocols in Molecular Biology, John Wiley & Sons, Inc., New York, 9.50-9.51, 11.7-11.8). For example, the stringent conditions may include conditions under which genes having a high homology or identity of 70% or more, 75% or more, 80% or more, 85% or more, 90% or more, 95% or more, 96% or more, 97% or more, 98% or more, or 99% or more are hybridized with each other and genes having a homology or identity lower than the above homologies or identities are not hybridized with each other, or washing conditions of Southern hybridization, that is, washing once, specifically twice or three times at a salt concentration and a temperature corresponding to 60°C, 1 × SSC, 0.1% SDS, specifically 60°C, 0.1 × SSC, 0.1% SDS, and more specifically 68°C, 0.1× SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present application may include isolated nucleotide fragments complementary to the entire sequence as well as nucleic acid sequences substantially similar thereto.

Specifically, polynucleotides having a homology or identity with the polynucleotide of the present application may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g*., Sambrook *et al*.).

Specifically, the *O*-phosphoserine-producing microorganism of the present application may be a microorganism in which the activity of erythronate-4-phosphate dehydrogenase is not inactivated, but is not limited thereto.

More specifically, the *O*-phosphoserine-producing microorganism of the present application may be a microorganism in which the polynucleotide encoding erythronate-4-phosphate dehydrogenase is not deleted, but is not limited thereto.

For the purpose of the present application, the microorganism may be one in which the activity of erythronate-4-phosphate dehydrogenase is weakened compared to its endogenous activity by including an expression vector for expressing the modified polynucleotide in a host cell, but is not limited thereto.

The vector of the present application may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome binding site, and a sequence for regulating termination of transcription and translation. Once transformed into a suitable microorganism, the vector may replicate or function independently from the host genome, or may integrate into genome thereof.

The vector used in the present application is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, Charon21A, *etc.* may be used; and as a plasmid vector, those based on pDZ, pBR, pUC, pBluescriptII, pGEM, pTZ, pCL pSK, pSKH, pET, *etc.* may be used. Specifically, pDZ, pDC, pDCM2, pACYC177, pACYC184, pCL, pSK, pSKH130, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target variant of the present application.

The modification of a part or all of the polynucleotide in the microorganism of the present application may be achieved by (a) homologous recombination using a vector for chromosomal insertion in the microorganism or genome editing using engineered nuclease (*e.g*., CRISPR-Cas9) and/or (b) may be induced by light, such as ultraviolet rays and radiation, *etc.,* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

For example, various methods known in the art may be used to substitute the initiation codons of target genes with TTG or CTG on the chromosome of the microorganism of the present application. For example, the initiator codon sequence of *pdxB* inherent in the microorganism may be substituted on the chromosome, or alternatively, the corresponding gene, in which the initiator codon sequence has been substituted, may be introduced into the microorganism in the form of a plasmid.

The O-phosphoserine-producing microorganism of the present application may further have enhanced YhhS protein activity compared to its endogenous activity.

As used herein, the term "YhhS" refers to a polypeptide exhibiting an O-phosphoserine-exporting activity, specifically refers to a membrane protein which has the activity of exporting OPS to the outside of the cell. In the present application, the YhhS may be a YhhS major facilitator superfamily (MFS) transporter having has the activity of exporting OPS to the outside of the cell. The YhhS has been identified as a protein exhibiting an OPS-exporting activity in *E. coli*, in which growth inhibition is released in a condition where an excessive OPS is present.

Specifically, the YhhS may be used interchangeably with the YhhS MFS transporter. In the present application, the amino acid sequence of the YhhS can be obtained from GenBank of the NCBI, a known database. The YhhS of the present application may be a protein having or including an amino acid sequence represented by SEQ ID NO: 7, or may be a protein consisting or consisting essentially of an amino acid sequence represented by SEQ ID NO: 7, but is not limited thereto. Additionally, the YhhS of the present application may have or include an amino acid sequence having a sequence homology or identity of 70%, 80%, 90%, 95%, or 99% or higher to the amino acid sequence represented by SEQ ID NO: 7, as long as it shows the YhhS activity. Moreover, the YhhS of the present application may consist or consist essentially of an amino acid sequence having a homology or identity of 70%, 80%, 90%, 95%, or 99% or higher to the amino acid sequence represented by SEQ ID NO: 7, but is not limited thereto. In addition, the polynucleotide encoding the YhhS may have or include a nucleotide sequence encoding the amino acid sequence represented by SEQ ID NO: 8. Further, the polynucleotide encoding the YhhS may consist or consist essentially of a nucleotide sequence encoding the amino acid sequence represented by SEQ ID NO: 7. The polynucleotide encoding YhhS of the present application may undergo various modifications in the coding region within the scope that does not change the amino acid sequence of the YhhS protein, due to codon degeneracy or in consideration of the codons preferred in an organism in which the YhhS protein is to be expressed. The polynucleotide encoding YhhS of the present application may have or include a nucleotide sequence having a homology or identity of 70%, 80%, 90%, 95%, or 99% or higher, and less than 100% to the nucleotide sequence of SEQ ID NO: 8. Additionally, the polynucleotide encoding YhhS of the present application may consist or consist essentially of a nucleotide sequence having a homology or identity of 70%, 80%, 90%, 95%, or 99% or higher, and less than 100% to the nucleotide sequence of SEQ ID NO: 8, but is not limited thereto.

The O-phosphoserine-producing microorganism of the present application may be one in which a protein activity of YhhS, phosphoserine aminotransferase (SerC), or a combination thereof is further introduced or enhanced.

The SerC has the activity of converting 3-phospho-hydroxypyruvate into *O-*phosphoserine, and thus, the microorganism introduced with SerC activity or modified to enhance the SerC activity has the property of accumulating OPS therein, thereby being useful for the production of OPS. The SerC of the present application may be a protein having or including an amino acid sequence represented by SEQ ID NO: 9, or may be a protein consisting or consisting essentially of an amino acid sequence represented by SEQ ID NO: 9, but is not limited thereto. Additionally, the SerC of the present application may have or include an amino acid sequence having a sequence homology or identity of 70%, 80%, 90%, 95%, or 99% or higher to the amino acid sequence represented by SEQ ID NO: 9, as long as it shows the SerC activity. Moreover, the SerC of the present application may consist or consist essentially of an amino acid sequence having a homology or identity of 70%, 80%, 90%, 95%, or 99% or higher to the amino acid sequence represented by SEQ ID NO: 9, but is not limited thereto. In addition, the polynucleotide encoding the SerC may have or include a nucleotide sequence encoding the amino acid sequence represented by SEQ ID NO: 10. Further, the polynucleotide encoding the SerC may consist or consist essentially of a nucleotide sequence encoding the amino acid sequence represented by SEQ ID NO: 9. The polynucleotide encoding SerC of the present application may undergo various modifications in the coding region within the scope that does not change the amino acid sequence of the SerC protein, due to codon degeneracy or in consideration of the codons preferred in an organism in which the SerC protein is to be expressed. The polynucleotide encoding SerC of the present application may have or include a nucleotide sequence having a homology or identity of 70%, 80%, 90%, 95%, or 99% or higher, and less than 100% to the nucleotide sequence of SEQ ID NO: 10. Additionally, the polynucleotide encoding SerC of the present application may consist or consist essentially of a nucleotide sequence having a homology or identity of 70%, 80%, 90%, 95%, or 99% or higher, and less than 100% to the nucleotide sequence of SEQ ID NO: 10, but is not limited thereto.

The O-phosphoserine-producing microorganism of the present application may be one in which an activity of phosphoserine phosphatase (SerB) is further weakened compared to its endogenous activity.

The SerB has the activity of converting O-phosphoserine to L-serine, and thus the microorganism modified to weaken the SerB activity has the property of accumulating OPS therein, thereby being useful for the production of OPS. The SerB of the present application may be a protein having or including an amino acid sequence represented by SEQ ID NO: 5, or may be a protein consisting or consisting essentially of an amino acid sequence represented by SEQ ID NO: 5, but is not limited thereto. Additionally, the SerB of the present application may have or include an amino acid sequence having a sequence homology or identity of 70%, 80%, 90%, 95%, or 99% or higher to the amino acid sequence represented by SEQ ID NO: 5, as long as it shows the SerB activity. Moreover, the SerB of the present application may consist or consist essentially of an amino acid sequence having a homology or identity of 70%, 80%, 90%, 95%, or 99% or higher to the amino acid sequence represented by SEQ ID NO: 5, but is not limited thereto. In addition, the polynucleotide encoding the SerB may have or include a nucleotide sequence encoding the amino acid sequence represented by SEQ ID NO: 6. Further, the polynucleotide encoding the SerB may consist or consist essentially of a nucleotide sequence encoding the amino acid sequence represented by SEQ ID NO: 5. The polynucleotide encoding SerB of the present application may undergo various modifications in the coding region within the scope that does not change the amino acid sequence of the SerB protein, due to codon degeneracy or in consideration of the codons preferred in an organism in which the SerB protein is to be expressed. The polynucleotide encoding SerB of the present application may have or include a nucleotide sequence having a homology or identity of 70%, 80%, 90%, 95%, or 99% or higher, and less than 100% to the nucleotide sequence of SEQ ID NO: 6. Additionally, the polynucleotide encoding SerB of the present application may consist or consist essentially of a nucleotide sequence having a homology or identity of 70%, 80%, 90%, 95%, or 99% or higher, and less than 100% to the nucleotide sequence of SEQ ID NO: 6, but is not limited thereto.

As used herein, the term "enhancement" of a polypeptide activity means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide can be confirmed by the increase in the level of activity of the polypeptide, expression level, or the amount of product excreted from the polypeptide.

The enhancement of the activity of the polypeptide can be applied by various methods well known in the art, and is not limited as long as it can enhance the activity of the target polypeptide compared to that of the microorganism before modification. Specifically, genetic engineering and/or protein engineering well known to those skilled in the art, which is a common method of molecular biology, may be used, but the method is not limited thereto (*e.g*., Sitnicka et al. Functional Analysis of Genes. Advances in Cell Biology. 2010, Vol. 2. 1-16; Sambrook et al. Molecular Cloning 2012, *etc*.).

Specifically, the enhancement of the polypeptide of the present application may be achieved by:
1) increasing the intracellular copy number of a polynucleotide encoding the polypeptide;
2) modifying the expression regulatory region of a gene encoding the polypeptide on the chromosome (*e.g*., inducing a modification on the expression regulatory region, replacing the sequence with a sequence having a stronger activity, or insertion of a sequence having a stronger activity);
3) modifying a nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

More specifically:
The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying a nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or polynucleotide sequence modified to have a stronger activity, or an amino acid sequence or polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration of the corresponding polypeptide is increased relative to the activity or concentration of the polypeptide expressed in a wild-type or a microorganism before modification, or that the amount of product produced from the polypeptide is increased, but is not limited thereto.

Additionally, the microorganism may be a microorganism in which its capability to introduce OPS into a cell or decompose OPS is further weakened.

Regarding the contents of the OPS-producing microorganism, the disclosures in US 8557549 B2 or U.S. Patent Application Publication No. 2012-0190081, *etc.* may be used as references of the present application, in addition to those described above, but the references are not limited thereto.

Another aspect of the present application provides a method for producing *O-*phosphoserine, including culturing the microorganism of the present application in a medium.

The method for producing *O*-phosphoserine of the present application may include culturing the microorganism of the present application in a medium.

As used herein, the term "cultivation" means that the microorganism of the present application is grown under appropriately controlled environmental conditions. The cultivation process of the present application may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

As used herein, the term "medium" refers to a mixture of materials which contains nutrient materials required for the cultivation of the microorganism of the present application as a main ingredient, and it supplies nutrient materials and growth factors, along with water that is essential for survival and growth. Specifically, the medium and other culture conditions used for culturing the microorganism of the present application may be any medium used for conventional cultivation of microorganisms without any particular limitation. However, the microorganism of the present application may be cultured under aerobic conditions in a conventional medium containing an appropriate carbon source, nitrogen source, phosphorus source, inorganic compound, amino acid, and/or vitamin, while adjusting temperature, pH, *etc.*

In the present application, the carbon source may include carbohydrates, such as glucose, saccharose, lactose, fructose, sucrose, maltose, *etc.*; sugar alcohols, such as mannitol, sorbitol, *etc.*; organic acids, such as pyruvic acid, lactic acid, citric acid, *etc.*; amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e*., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.*; amino acids, such as glutamic acid, methionine, glutamine, *etc.*; and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compound may include sodium chloride, calcium chloride, iron chloride, magnesium sulfate, iron sulfate, manganese sulfate, calcium carbonate, *etc.* Additionally, amino acids, vitamins, and/or appropriate precursors may be included. These constituting ingredients or precursors may be added to a medium in a batch or continuous manner, but these phosphorus sources are not limited thereto.

Additionally, the medium may include metal salts, such as magnesium sulfate or iron sulfate, and may further contain amino acids, vitamins and appropriate precursors. These media or precursors may be added to the culture in a batch or continuous manner, but are not limited thereto.

In one example, for the cultivation of the recombinant microorganism having weakened SerB activity compared to its endogenous activity, the medium may further contain glycine or serine, as the serine requirement of the recombinant microorganism is induced. Glycine may be provided in the form of purified glycine, a glycine-containing yeast extract, or tryptone. The concentration of glycine to be contained in the medium is generally 0.1 g/L to 10 g/L, and specifically 0.5 g/L to 3 g/L. Additionally, serine may be provided in the form of purified serine, a serine-containing yeast extract, or tryptone. The concentration of serine to be contained in the medium is generally 0.1 g/L to 5 g/L, and specifically 0.1 g/L to 1 g/L.

Additionally, the pH of the medium may be adjusted by adding a compound such as ammonium hydroxide, potassium hydroxide, ammonia, phosphoric acid, sulfuric acid, *etc.* during the cultivation of the microorganism of the present application in an appropriate manner. Additionally, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature in the cultivation of the present application may be in the range from 20°C to 45°C and specifically from 25°C to 40°C. The cultivation may be carried out for about 10 to 160 hours, but is not limited thereto.

The *O*-phosphoserine produced by the cultivation of the present application may be released into the medium or remain in the cells.

The method for producing *O*-phosphoserine of the present application may further include a step of preparing the microorganism of the present application, a step of preparing a medium for culturing the microorganism, or a combination thereof (regardless of the order, in any order), for example, prior to the culturing step.

The method for producing *O*-phosphoserine of the present application may further include a step of recovering *O*-phosphoserine from the culture medium (medium on which the culture was grown) or the microorganism of the present application. The recovering step may be further included after the culturing step.

In the recovering step, desired *O*-phosphoserine may be collected using the method of culturing a microorganism of the present application, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC or a combination thereof may be used, and the desired *O*-phosphoserine can be recovered from the medium or the microorganisms using suitable methods known in the art.

Further, the method for producing *O*-phosphoserine of the present application may further include a purification step, which may be performed using an appropriate method known in the art. In one example, when the method for producing *O*-phosphoserine of the present application includes both a recovering step and a purification step, the recovering step and the purification step may be performed continuously or intermittently regardless of the order or simultaneously, or may be integrated into one step, but the method is not limited thereto.

In the method of the present application, the "microorganism", "*O-*phosphoserine", *etc.* are the same as described in other aspects.

Still another aspect of the present application provides a method for producing cysteine or a derivative thereof, including: a) culturing the microorganism of the present application in a medium to produce *O*-phosphoserine or a medium containing the same; and b) reacting O-phosphoserine sulfhydrylase (OPSS) or a microorganism expressing the same; O-phosphoserine produced in step a) or a medium containing the same; and a sulfide.

In the method of the present application, the "microorganism", "*O-*phosphoserine", *etc.* are the same as described in other aspects.

Specifically, the method may be a method for producing cysteine or a derivative thereof: including culturing an *O*-phosphoserine-producing microorganism, in which an activity of erythronate-4-phosphate dehydrogenase is weakened compared to its endogenous activity, in a medium to produce *O*-phosphoserine or a medium containing the same; and reacting O-phosphoserine sulfhydrylase (OPSS) or a microorganism expressing the same; O-phosphoserine produced in step a) or a medium containing the same; and a sulfide.

As used herein, the term "derivative" refers to similar compounds obtained by chemically modifying a portion of any compound. The term usually refers to compounds in which a hydrogen atom or a particular atom group is substituted with another atom or atom group.

As used herein, the term "cysteine derivative" refers to compounds in which a hydrogen atom or a particular atom group in cysteine is substituted with another atom or atom group. For example, the cysteine derivatives may have a form in which the nitrogen atom of the amine group (-NH₂) or the sulfur atom of the thiol group (-SH) in cysteine has another atom or atom group attached thereto, and the examples of cysteine derivatives may include NAC (N-acetylcysteine), SCMC (S-carboxymethylcysteine), Boc-Cys(Me)-OH, (R)-S-(2-amino-2-carboxyethyl)-L-homocysteine, (*R*)-2-amino-3-sulfopropionic acid, D-2-amino-4-(ethylthio)butyric acid, 3-sulfino-L-alanine, Fmoc-Cys(Boc-methyl)-OH, seleno-L-cystine, *S*-(2-thiazolyl)-L-cysteine, *S*-(2-thienyl)-L-cysteine, *S*-(4-tolyl)-L-cysteine, *etc.,* but are not limited thereto.

As long as cysteine is produced according to the method of the present application, conversion to cysteine derivatives can be easily performed by converting cysteine into various cysteine derivatives by a method well known in the art.

Specifically, the method of producing cysteine derivatives may further include converting the cysteine produced in step b) into a cysteine derivative. For example, cysteine may be synthesized into *N*-acetylcysteine (NAC) by a reaction with an acetylation agent, or it may be synthesized into *S*-carboxymethylcysteine (SCMC) by a reaction with a haloacetic acid in basic conditions, but is not limited thereto.

These cysteine derivatives are used mainly as pharmaceutical materials for antitussive agents, cough-relieving agents, and therapeutic agents for bronchitis, bronchial asthma, laryngopharyngitis, *etc.,* but are not limited thereto.

As used herein, the term "*O*-phosphoserine sulfhydrylase (OPSS)" refers to an enzyme that catalyzes a reaction by which OPS is converted into cysteine by providing a thiol group (SH group) to OPS. The enzyme may have been first found in *Aeropyrum pernix*, *Mycobacterium tuberculosis*, *Mycobacterium smegmatics*, and *Trichomonas vaginalis* (Mino K. and Ishikawa K., FEBS Letters, 551:133-138, 2003; Burns, K. E. et al., J. Am. Chem. Soc., 127:11602-11603, 2005). Additionally, the OPSS may include not only wild-type OPSS, but also variants that include deletion, substitution, or addition in part of the polynucleotide sequence encoding the OPSS which show activity that is equal to or higher than the biological activity of the wild-type OPSS, and may also include all of OPSS disclosed in US 8557549 B2 and US 9127324 B2 and their variants.

The sulfide may be any sulfide provided not only in a solid form generally used in the art, but also in a liquid or gas form due to the difference in pH, pressure, and solubility, and thus can be converted to a thiol (SH) group in the form of, for example, sulfide (S²⁻) or thiosulfate (S₂O₃²⁻) without limitation. Specifically, the sulfide may include Na₂S, NaSH, H₂S, (NH₄)₂S, and Na₂S₂O₃, which can provide a thiol group to OPS, but is not limited thereto. In the reaction, a single thiol group is provided to a single reactive OPS group to produce a single cysteine or a derivative thereof. In this reaction, a sulfide is specifically added in an amount of 0.1 to 3 molar equivalents, and specifically 1 to 2 molar equivalents based on the molar concentration of OPS, but is not limited thereto.

Further, the method of the present application may further include a step of recovering the cysteine produced in the above reaction step. In particular, the desired cysteine may be recovered by separation and purification from the reaction solution using a suitable reaction known in the art.

Yet another aspect of the present application provides a composition for producing O-phosphoserine, including the microorganism of the present application, in which the activity of erythronate-4-phosphate dehydrogenase is weakened compared to its endogenous activity.

The composition of the present application may further include any suitable excipient commonly used in compositions for producing *O*-phosphoserine, such excipients include, for example, preservatives, wetting agents, dispersing agents, suspending agents, buffers, stabilizers, or isotonic agents, *etc.,* but are not limited thereto.

In the composition of the present application, the "microorganism", "*O-*phosphoserine", *etc.* are the same as described in other aspects.

Even another aspect of the present application provides the use of the microorganism of the present application, in which the activity of erythronate-4-phosphate dehydrogenase is weakened compared to its endogenous activity, in the production of *O*-phosphoserine, cysteine, or cysteine derivatives.

### [Mode for Carrying Out the Invention]

Hereinafter, the present application will be described in detail by way of Examples. However, these Examples are merely preferred Examples given for illustrative purposes, and thus, the scope of the present application is not intended to be limited to or by these Examples. Meanwhile, technical features which are not described herein can be sufficiently understood and easily carried out by those skilled in the art in the technical field of the present application or in a similar technical field.

### Example 1: Construction of Strain with Enhanced YhhS Expression and Evaluation of OPS Producing Ability

### 1-1. Construction of Plasmid for Enhancing yhhS Expression

The gene fragments in the upstream region of the *yhhS* gene wild-type promoter where homologous recombination occurs on chromosomes were obtained using a primer pair of SEQ ID NOS: 11 and 12 based on the wild-type *E. coli* ATCC27325 chromosomal DNA as a template. In addition, the Ptrc promoter was obtained using a primer pair of SEQ ID NOS: 13 and 14 based on the pCL_Ptrc-gfp (WO 2016024771 A1) as a template. Further, the gene fragments in the downstream region of the *yhhS* gene wild-type promoter where homologous recombination occurs on chromosomes were obtained using a primer pair of SEQ ID NOS: 15 and 16 based on the *E*. *coli* ATCC27325 chromosomal DNA as a template. The primer sequences used are shown in Table 1 below.

**[Table 1]**

| SEQ ID NO: | Name | Sequence (5'→3') |
|---|---|---|
| 11 | yhhS UP F | CTGCAGGAATTCGATGAAGGTAACCACTTTTTCCG |
| 12 | yhhS UP R | GAGTTGCAGCAAGCGGAGGATCACCACATTTTTAC |
| 13 | Ptrc F | AATGTGGTGATCCTCCGCTTGCTGCAACTCTCTCA |
| 14 | Ptrc R | TACGGGTTCGGGCATGATAGCTCTCCTGTGTGAAA |
| 15 | yhhS Down F | CACAGGAGAGCTATCATGCCCGAACCCGTAGCCGA |
| 16 | yhhS Down R | GTCGACTAGCGTGATAGCGGTTTGCCTTTACTGGC |

In order to obtain the fragments, PCR was performed using Solg^{™} Pfu-X DNA polymerase, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments of the *yhhS* promoter, the Ptrc promoter fragment, and the downstream fragments of the *yhhS* promoter obtained by the above process were cloned with the pSKH130 vector for chromosomal transformation cleaved with the restriction enzyme EcoRV using an in-fusion cloning kit (Clontech Laboratories, Inc.) to obtain a recombinant plasmid, which was named pSKH_Ptrc-yhhS. The pSKH130 vector is a vector containing a PI protein (pir gene)-dependent R6K replicon, SacB (Levansucrase) gene, and kanamycin resistance gene.

### 1-2. Construction of Strain with Enhanced YhhS Expression

In order to further improve the OPS exporting ability of OPS, the expression of YhhS (SEQ ID NO: 7), a protein having an OPS exporting ability, was enhanced based on CA07-0012 (KCCM11121P, US 8557549 B2), which is a strain in which the endogenous phosphoserine phosphatase (SerB) is deleted.

Specifically, the pSKH_Ptrc-yhhS prepared in Example 1-1 was transformed into the CA07-0012, which is a host with weakened OPS decomposing ability, by electroporation (Appl. Microbiol. Biotechnol. (1999) 52:541-545). After obtaining the desired strain using R6K and kanamycin at the first crossover, the strain was subjected to secondary crossover in a medium containing sucrose to obtain a strain from which the kanamycin resistance gene was deleted and into which the Ptrc promoter nucleotide sequence was inserted at the terminal of the wild-type promoter nucleotide sequence of the *yhhS* gene. The insertion of the Ptrc promoter nucleotide sequence was confirmed through genome sequencing and PCR amplification using a primer pair of SEQ ID NO: 17 and SEQ ID NO: 18, which can respectively amplify the external region of the upstream region and downstream region of the corresponding homologous recombination. The strain (CA07-0012::Ptrc-yhhS) into which the promoter sequence was inserted based on CA07-0012 as a host was named CA07-4821.

**[Table 2]**

| SEQ ID NO: | Name | Sequence (5'→3') |
|---|---|---|
| 17 | yhhSP Conf F | AGTTGAGCAAAAACGCCAGT |
| 18 | yhhSP Conf R | GCCATACCGTACAGCCAGAC |

### 1-3. Evaluation of OPS Producing Ability of Strain with Enhanced YhhS Expression

In order to evaluate the O-phosphoserine producing ability of the strain with enhanced YhhS expression prepared in Example 1-2, the following medium (Table 3) was used.

**[Table 3]**

| Medium Component | Amount |
|---|---|
| Glucose | 40 g |
| KH₂PO₄ (KP1) | 6 g |
| (NH₄)₂SO₄ | 17g |
| MgSO₄·7H₂O | 1 g |
| MnSO₄·4H₂O | 5 mg |
| FeSO₄·7H₂O | 10 mg |
| L-Glycine | 1.5 g/L |
| Yeast Extract | 2.5 g/L |
| CaCO₃ | 30 g/L |
| pH | 6.8 |

Specifically, each of the strains was plated out on a solid LB medium and cultured in a 33°C incubator overnight. The strains cultured in the solid LB medium overnight were inoculated into a 25 mL titer medium shown in Table 3 below and then cultured in an incubator at a rate of 200 rpm for 48 hours at 33°C. The results are shown in Table 4.

**[Table 4]**

| Name of Strain | OPS (g/L) |
|---|---|
| CA07-0012 | 1.95 |
| CA07-4821(CA07-0012::Ptrc-yhhS) | 2.47 |

As shown in Table 4, it was confirmed that the OPS producing ability of CA07-4821, a strain with enhanced YhhS expression, was increased by about 127% compared to the parent strain.

### Example 2: Construction of Strain with Enhanced SerC Expression and Evaluation of OPS Producing Ability

### 2-1. Construction of Plasmid for Enhancing SerC Expression

In order to insert the endogenous *serC* gene and its promoter into the maeB position on the host genome, the gene fragments in the upstream region of the maeB gene wild-type promoter where homologous recombination occurs on chromosomes were obtained using a primer pair of SEQ ID NOS: 19 and 20 based on the wild-type *E. coli* ATCC27325 chromosomal DNA as a template. In addition, the *serC* gene and its wild-type promoter region were obtained using a primer pair of SEQ ID NOS: 21 and 22 based on the wild-type *E. coli* ATCC27325 chromosomal DNA as a template. Further, the gene fragments in the downstream region of the maeB gene wild-type promoter where homologous recombination occurs on chromosomes were obtained using a primer pair of SEQ ID NOS: 23 and 24 based on the wild-type *E*. *coli* ATCC27325 chromosomal DNA as a template.

The primer sequences used are shown in Table 5 below.

**[Table 5]**

| SEQ ID NO: | Name | Sequence (5'→3') |
|---|---|---|
| 19 | maeB UP F1 | CCGGGCTGCAGGAATTCGATCTTCCCAGGTCGAAGCCAGC |
| 20 | maeB UP R1 | CATATGAATGATTTCACTACAGGTTGTTCCTTTCACGTAA |
| 21 | Pn_serC F | TTACGTGAAAGGAACAACCTGTAGTGAAATCATTCATATG |
| 22 | Pn_serC R | ATTCAGGGTAAGCGTGAGAGTTAACCGTGACGGCGTTCGA |
| 23 | maeB | CGAACGCCGTCACGGTTAACTCTCACGCTTACCCTGAAT |
| | Down F1 | |
| 24 | maeB Down R1 | GCTAGGTCGACTAGCGTGATCCCTGCAACGCATTAAATAA |

In order to obtain the fragments, PCR was performed using Solg^{™} Pfu-X DNA polymerase, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments of the *maeB* gene region, the fragment of the *serC* gene region containing the wild-type promoter, and the downstream fragments of the maeB promoter obtained by the above process were cloned with the pSKH130 vector for chromosomal transformation cleaved with the restriction enzyme EcoRV using an in-fusion cloning kit (Clontech Laboratories, Inc.) to obtain a recombinant plasmid, which was named pSKHΔmaeB::Pn_serC.

A plasmid containing the nucleotide sequence for the *maeB* deletion was constructed in order to investigate the effect of the deleted *maeB* gene on the OPS producing ability for the additional introduction of the *serC* gene into the host genome. For the deletion of *maeB* gene, the gene fragments in the upstream region of the *maeB* gene wild-type promoter where homologous recombination occurs on chromosomes were obtained using a primer pair of SEQ ID NOS: 25 and 26 based on the wild-type *E*. *coli* ATCC27325 chromosomal DNA as a template. In addition, the gene fragments in the downstream region of the *maeB* gene wild-type promoter where homologous recombination occurs on chromosomes were obtained using a primer pair of SEQ ID NOS: 27 and 28 based on the *E. coli* ATCC27325 chromosomal DNA as a template.

The primer sequences used are shown in Table 6 below.

**[Table 6]**

| SEQ ID NO: | Name | Sequence (5'→3') |
|---|---|---|
| 25 | ΔmaeB UP F | CTGCAGGAATTCGATCTTCCCAGGTCGAAGCCAGC |
| 26 | ΔmaeB UP R | ATTCAGGGTAAGCGTGAGAGGGTTGTTCCTTTCACGTAA |
| 27 | ΔmaeB Down F | TTACGTGAAAGGAACAACCCTCTCACGCTTACCCTGAAT |
| 28 | ΔmaeB Down R | GTCGACTAGCGTGATCCCTGCAACGCATTAAATAA |

In order to obtain the fragments, PCR was performed using Solg^{™} Pfu-X DNA polymerase, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments of the *maeB* gene region and the downstream fragments of the *maeB* promoter obtained by the above process were cloned with the pSKH130 vector for chromosomal transformation cleaved with the restriction enzyme EcoRV using an in-fusion cloning kit (Clontech Laboratories, Inc.) to obtain a recombinant plasmid, which was named pSKHΔmaeB.

### 2-2. Construction of Strain with Enhanced SerC Expression

The pSKHΔmaeB::Pn_serC prepared in Example 2-1 was transformed into the CA07-4821 prepared in Example 1-2 by electroporation (Appl. Microbiol. Biotechnol. (1999) 52:541-545), and then subjected to secondary crossover to obtain a strain into which the *serC* gene nucleotide sequence including the wild-type promoter was inserted at the position of the *maeB* gene nucleotide sequence. The insertion of the *serC* gene nucleotide sequence including the wild-type promoter was confirmed through genome sequencing and PCR amplification using a primer pair of SEQ ID NO: 29 and SEQ ID NO: 30, which can respectively amplify the external region of the upstream region and downstream region of the corresponding homologous recombination. The strain into which the nucleotide sequence was inserted based on CA07-4821 of Example 1-2 as a host was named CA07-4881.

In addition, in order to delete mae8 on the host genome, the pSKHΔmaeB prepared in Example 2-1 was transformed into the CA07-4821 prepared in Example 1-2 by electroporation (Appl. Microbiol. Biotechnol. (1999) 52:541-545), and then subjected to secondary crossover to obtain a strain from which the *maeB* gene nucleotide sequence was deleted. The deletion of the *maeB* gene nucleotide sequence was confirmed through genome sequencing and PCR amplification using a primer pair of SEQ ID NO: 29 and SEQ ID NO: 30, which can respectively amplify the external region of the upstream region and downstream region of the corresponding homologous recombination. The strain from which the *maeB* gene nucleotide sequence was deleted based on CA07-4821 of Example 1-2 as a host was named CA07-4882.

The primer sequences used are shown in Table 7 below.

**[Table 7]**

| SEQ ID NO: | Name | Sequence (5'→3') |
|---|---|---|
| 29 | maeB out conf F | AAAACGCCTGCTTCCGCAC |
| 30 | maeB out conf R | CGTGGTAATTAAGGCGTGAT |

### 2-3. Evaluation of OPS Producing Ability of Strain with Enhanced SerC Expression

In order to evaluate the O-phosphoserine producing ability of the CA07-4881 and CA07-4882 prepared in Example 2-2, the control CA07-0012, and CA07-4821, the medium of Example 1-3 (Table 3) was used.

Specifically, each of the strains was plated out on a solid LB medium and cultured in a 33°C incubator overnight. The strains cultured in the solid LB medium overnight were inoculated into a 25 mL titer medium shown in Table 3 below and then cultured in an incubator at a rate of 200 rpm for 48 hours at 33°C. The results are shown in Table 8.

**[Table 8]**

| Name of Strains | OPS (g/L) |
|---|---|
| CA07-0012 | 1.95 |
| CA07-4821 (CA07-0012::Ptrc-yhhS) | 2.47 |
| CA07-4881(CA07-4821 ΔmaeB::Pn_serC) | 3.55 |
| CA07-4882(CA07-4821ΔmaeB) | 2.44 |

As shown in Table 8, in the case of CA07-4881, in which *serC* was enhanced through the additional introduction of *serC* into the host genome, it was confirmed that the OPS producing ability was increased by about 144% compared to the parent strain CA07-4821. Meanwhile, it was confirmed that CA07-4882, a strain with deleted *maeB*, showed no difference in OPS producing ability compared to the parent strain (CA07-4821).

### Example 3: Construction of Strains with Weakened or Deleted pdxB Expression and Evaluation of OPS Producing Ability

### 3-1. Construction of Plasmids for Weakening or Deletion of pdxB Expression

In order to reduce the expression level of the *pdxB* gene, the initiation codon of the *pdxB* gene was replaced, or a plasmid for deletion of the *pdxB* gene was constructed.

Specifically, the initiation codon sequence (gtg) of *pdxB* (SEQ ID NO: 2) was replaced with ttg to ctg. First, in order to replace the initiation codon sequence (gtg) with ttg, the gene fragments in the upstream region including the mutation (ttg) on the wild-type initiation codon sequence of the *pdxB* gene where homologous recombination occurs on chromosomes were obtained using a primer pair of SEQ ID NOS: 31 and 32 based on the wild-type *E*. *coli* ATCC27325 chromosomal DNA as a template.

In addition, the gene fragments in the downstream region including the mutation (ttg) on the wild-type initiation codon sequence of the *pdxB* gene where homologous recombination occurs on chromosomes were obtained using a primer pair of SEQ ID NOS: 33 and 34 based on the wild-type *E. coli* ATCC27325 chromosomal DNA as a template.

Next, in order to replace the initiation codon sequence (gtg) with ctg, the gene fragments in the upstream region including the mutation (ctg) on the wild-type initiation codon sequence of the *pdxB* gene where homologous recombination occurs on chromosomes were obtained using a primer pair of SEQ ID NOS: 31 and 35 based on the *E*. *coli* ATCC27325 chromosomal DNA as a template.

In addition, the gene fragments in the downstream region including the mutation (ctg) on the wild-type initiation codon sequence of the *pdxB* gene where homologous recombination occurs on chromosomes were obtained using a primer pair of SEQ ID NOS: 36 and 34 based on the *E*. *coli* ATCC27325 chromosomal DNA as a template.

Meanwhile, in order to delete *pdxB* gene, the gene fragments in the upstream region of the *pdxB* gene where homologous recombination occurs on chromosomes were obtained using a primer pair of SEQ ID NOS: 37 and 38 based on the *E. coli* ATCC27325 chromosomal DNA as a template. In addition, the gene fragments in the downstream region of the *pdxB* gene where homologous recombination occurs on chromosomes were obtained using a primer pair of SEQ ID NOS: 39 and 40 based on the *E*. *coli* ATCC27325 chromosomal DNA as a template.

The primer sequences used are shown in Table 9 below. Specifically, in the names of SEQ ID NO: 31 and SEQ ID NO: 34, n refers to t or c.

**[Table 9]**

| SEQ ID NO: | Name | Sequence (5'→3') |
|---|---|---|
| 31 | pdxB(ntg)U p F | CTGCAGGAATTCGATGCAACAGGGTGAGAATGG TT |
| 32 | pdxB(ttg)Up R | AACAAGGATTTTCAAGTTTGTGTTACCTGTATGA GACG |
| 33 | pdxB(ttg)Do wn F | CAGGTAACACAAACTTGAAAATCCTTGTTGATGA AAAT |
| 34 | pdxB(ntg)D own R | GTCGACTAGCGTGATTGGCGTATGGAAAGTCAG AA |
| 35 | pdxB(ctg)Up R | AACAAGGATTTTCAGGTTTGTGTTACCTGTATGA GACG |
| 36 | pdxB(ctg)Do wn F | CAGGTAACACAAACCTGAAAATCCTTGTTGATGA AAAT |
| 37 | ΔpdxB Up F | CGGTGAGAGCTGATTCAGCG |
| 38 | ΔpdxB Up R | GTTTGTGTTACCTGTATGAG |
| 39 | ΔpdxB Down F | TCTCTTCTTCATGCTCTCTG |
| 40 | ΔpdxB Down R | GATTAACGGTTTCAGTGCCG |

In order to obtain the fragments, PCR was performed using Solg^{™} Pfu-X DNA polymerase, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments including the *pdxB* initiation codon mutations and the downstream fragments including the *pdxB* initiation codon mutations obtained by the above process were cloned with the pSKH130 vector for chromosomal transformation cleaved with the restriction enzyme EcoRV using an in-fusion cloning kit (Clontech Laboratories, Inc.) to obtain recombinant plasmids, which were named pSKH_pdxB(ttg) and pSKH_pdxB(ctg), respectively.

Meanwhile, the upstream fragments and the downstream fragments for *pdxB* deletion obtained by the above process were cloned with the pSKH130 vector for chromosomal transformation cleaved with the restriction enzyme EcoRV using an in-fusion cloning kit (Clontech Laboratories, Inc.) to obtain a recombinant plasmid, which was named pSKH ΔpdxB.

### 3-2. Construction of Strains with Weakened or Deleted pdxB Expression

The pSKH_pdxB(ttg) and pSKH_pdxB(ctg) prepared in Example 3-1 were transformed into the CA07-4881 prepared in Example 2-1 by electroporation (Appl. Microbiol. Biotechnol. (1999) 52:541-545), and then subjected to secondary crossover to obtain a strain in which the wild-type initiation codon sequence of the *pdxB* gene was substituted with ttg or ctg. The substitution of the nucleotide sequence of the *pdxB* initiation codon was confirmed through genome sequencing and PCR amplification using a primer pair of SEQ ID NO: 41 and SEQ ID NO: 42, which can respectively amplify the external region of the upstream region and downstream region of the corresponding homologous recombination. The strain in which the initiation codon sequence was replaced with ttg was named CA07-4883, and the strain in which the initiation codon sequence was replaced with ctg was named CA07-4884, based on CA07-4881 of Example 2-2 as a host.

Meanwhile, the pSKHΔpdxB(ttg) prepared in Example 3-1 was transformed into the CA07-4881 prepared in Example 2-1 by electroporation (Appl. Microbiol. Biotechnol. (1999) 52:541-545), and then subjected to secondary crossover to obtain a strain in which the *pdxB* gene was deleted. The deletion of the *pdxB* nucleotide sequence was confirmed through genome sequencing and PCR amplification using a primer pair of SEQ ID NO: 41 and SEQ ID NO: 42, which can respectively amplify the external region of the upstream region and downstream region of the corresponding homologous recombination. The strain in which the *pdxB* nucleotide sequence was deleted based on CA07-4881 of Example 2-2 as a host was named CA07-4886.

The primer sequences used are shown in Table 10 below.

**[Table 10]**

| SEQ ID NO: | Name | Sequence (5'→3') |
|---|---|---|
| 41 | pdxB Conf F | TCGATTGCCAGATCTGTTTG |
| 42 | pdxB Conf R | GACGTGCCGATATCCACTTT |

### 3-3. Evaluation of OPS Producing Ability of Strains with Weakened or Deleted pdxB Expression

In order to evaluate the *O*-phosphoserine producing ability of the CA07-4883, CA07-4884 and CA07-4886 strains prepared in Example 3-2, the control CA07-0012, CA07-4821, CA07-4881, and CA07-4882, the medium of Example 1-3 (Table 3) was used.

Specifically, each of the strains was plated out on a solid LB medium and cultured in a 33°C incubator overnight. The strains cultured in the solid LB medium overnight were inoculated into a 25 mL titer medium shown in Table 3 below and then cultured in an incubator at a rate of 200 rpm for 48 hours at 33°C. The results are shown in Table 11.

**[Table 11]**

| Name of Strains | OPS (g/L) |
|---|---|
| CA07-0012 | 1.95 |
| CA07-4821(CA07-0012::Ptrc-yhhS) | 2.47 |
| CA07-4881(CA07-4821 ΔmaeB::Pn_serC) | 3.55 |
| CA07-4882(CA07-4821 ΔmaeB) | 2.44 |
| CA07-4883(CA07-4881ΔpdxB::pdxB(ttg)) | 3.82 |
| CA07-4884(CA07-4881ΔpdxB::pdxB(ctg)) | 3.68 |
| CA07-4886(CA07-4881::ΔpdxB) | 3.25 |

As shown in Table 11, it was confirmed that the OPS producing ability of CA07-4883 and CA07-4884 in which the *pdxB* initiation codon was substituted with ttg or ctg, respectively, was increased by about 103% and 107% compared to the parent strain (CA07-4881). Meanwhile, it was confirmed that the OPS producing ability of CA07-4886, a strain with deleted *pdxB*, was lower than that of the parent strain (CA07-4881).

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. The scope of the present application is therefore indicated by the appended claims rather than by the foregoing description. All changes which come within the meaning and range of equivalency of the claims are to be embraced within the scope of the present application.

## Claims

1. An O-phosphoserine-producing microorganism, in which an activity of erythronate-4-phosphate dehydrogenase is weakened.

2. The microorganism of claim 1, wherein an initiation codon of a polynucleotide encoding the erythronate-4-phosphate dehydrogenase is substituted with TTG or CTG.

3. The microorganism of claim 2, wherein the microorganism has an increased O-phosphoserine productivity compared to a microorganism in which the initiation codon of a polynucleotide encoding the erythronate-4-phosphate dehydrogenase is GTG or ATG.

4. The microorganism of claim 1, wherein an YhhS protein activity is further enhanced.

5. The microorganism of claim 1, wherein an activity of phosphoserine phosphatase is further weakened.

6. The microorganism of claim 1, wherein a protein activity of YhhS, phosphoserine aminotransferase, or a combination thereof is introduced or enhanced.

7. The microorganism of claim 1, wherein the microorganism is a microorganism of the genus E*scherichia.*

8. The microorganism of claim 7, wherein the microorganism of the genus *Escherichia* is *E*. *coli.*

9. A method for producing O-phosphoserine, comprising culturing an O-phosphoserine-producing microorganism, in which an activity of erythronate-4-phosphate dehydrogenase is weakened, in a medium.

10. A method for producing cysteine or a derivative thereof, comprising:
a) culturing an O-phosphoserine-producing microorganism, in which an activity of erythronate-4-phosphate dehydrogenase is weakened, in a medium to produce O-phosphoserine or a medium containing the same; and
b) reacting O-phosphoserine sulfhydrylase (OPSS) or a microorganism expressing the same; O-phosphoserine produced in step a) or a medium containing the same; and a sulfide.
